# FASCICULE DE BREVET EUROPEEN

(11) **EP 1 820 526 B1**
(45) Date de publication et mention de la délivrance du brevet: **28.12.2011**
(21) Numéro de dépôt: 07368003.5
(22) Date de dépôt: 20.02.2007
(51) Int. Cl.: A61M 16/00

(54) **Procédé et appareil de distribution d'air**
Verfahren und Gerät zur Luftverteilung
Air distribution method and device

(30) Priorité: 20.02.2006 FR 0601449
(43) Date de publication de la demande: 22.08.2007
(73) Titulaire: Kaerys, 06300 Nice (FR)
(72) Inventeur: Delache, Alain, 06300 Nice (FR); Castelo, Véronique, 06300 Nice (FR)
(74) Mandataire: Damen, Daniel Martijn

(56) Documents cités:
- WO-A-2006/086190
- WO-A2-02/26283
- FR-A1- 2 726 191
- US-A1- 2006 005 834

## Description

L'invention concerne un appareil d'assistance respiratoire du type d'un appareil à distribution d'air sous pression positive continue, communément appelé PPC (pression positive continue), ou encore CPAP *(« continuous positive airway pressure* »), selon la terminologie anglaise.

Les appareils PPC connus de l'état de la technique sont utilisés notamment pour le traitement des apnées du sommeil. Dans le cas d'apnées du sommeil, ces dispositifs fournissent de même de l'air aux patients à une pression plus élevée que la pression atmosphérique de manière à éliminer les obstructions susceptibles de provoquer les apnées.

Certains appareils CPAP délivrent de l'air aux patients sous une pression constante dans le temps. Toutefois, la respiration sous une pression de traitement est relativement inconfortable en raison des pressions appliqués.

D'autres appareils sont aptes à délivrer de l'air sous deux pressions différentes, une première pression lors des phases inspiratoires et une seconde, lors des phases expiratoires. L'obstruction ayant lieu au début de la phase inspiratoire ou à la fin de la phase expiratoire, ces pompes appliquent une pression moins forte lors de l'expiration, permettant d'améliorer ainsi le confort du patient. Cependant, avec de tels appareils, la pression de traitement nécessaire à empêcher l'obstruction n'est pas encore atteinte lors du passage de la phase expiratoire à la phase inspiratoire, et une obstruction peut alors se produire à ce moment précis. Bien que plus confortables, ces appareils sont donc moins efficaces.

De plus, ces derniers appareils, de même que les appareils précités à pression constante, n'autorisent généralement pas un ajustement automatique de la pression lorsque surviennent des évènements respiratoires tels que des ronflements, des apnées ou des hypopnées.

D'autres appareils encore de l'art antérieur, cette fois plus perfectionnés, ajustent automatiquement la pression à appliquer aux voies aériennes supérieures des patients en fonction de la nature des évènements respiratoires. Ils comportent alors par exemple une unité de contrôle de pression comprenant préférentiellement un module destiné à estimer la pression de l'air à appliquer au patient en fonction du débit déterminé par les appareils. Toutefois, les pressions sont ajustées suite à la survenance effective des évènements. Ainsi, dans le cas des apnées obstructives, ces appareils réagissent tardivement en augmentant la pression à la prochaine inspiration, ou alors, en diminuant la pression lors de l'expiration suivant l'évènement. Dans ce dernier cas, la diminution de pression peut favoriser l'apparition d'apnées obstructives, qui se révèlent bien souvent en phase expiratoire. FR 2 726 191 montre les caractéristiques du préambule de la revendication 1.

Compte tenu de ce qui précède, un problème que se propose de résoudre l'invention est de réaliser un appareil qui permettent d'ajuster la pression à appliquer aux voies aériennes supérieures des patients en fonction de la nature des évènements respiratoires, l'ajustement étant effectué préalablement à la survenance effective desdits évènements. En particulier, dans le cas des apnées obstructives, l'appareil est apte à réagir en phase inspiratoire et non pas en phase expiratoire.

La solution de l'invention à ce problème a pour objet un appareil de distribution d'air au moyen d'un appareil d'assistance respiratoire à distribution d'air sous pression positive continue selon lequel on évalue un débit d'air de base et un débit d'air maximal pour un patient, et caractérisé en ce que on définit, pour ce patient, un débit d'air minimal à atteindre en phase inspiratoire, la valeur dudit débit d'air minimal à atteindre étant supérieure au débit de base et inférieure au débit maximal, de manière à ce que, lorsque le débit d'air mesuré par l'appareil n'atteint pas le débit d'air minimal à atteindre dans une phase inspiratoire, la pression d'air délivré par l'appareil au patient continue de croître et, lorsque le débit d'air atteint le débit d'air minimal à atteindre, dans ladite phase inspiratoire, cette pression décroît.

Préférentiellement, la valeur du débit d'air minimal à atteindre (Dₘᵢₙ(insp)) est comprise entre 70 et 90 % de la valeur du débit d'air maximal (Dₘₐₓ).

Préférentiellement, on définit une valeur seuil de débit d'air (Dₛₑᵤᵢₗ), cette valeur seuil étant supérieure à la valeur de base du débit d'air (D_{base}) et inférieure à la valeur du débit minimal à atteindre en phase inspiratoire, et en ce que, lorsque le débit d'air atteint cette valeur seuil en phase inspiratoire, la croissance de la pression d'air délivré (P) par l'appareil débute.

Préférentiellement, selon la présente invention, l'augmentation de pression (p_{bump}) est comprise entre 0,05 et 20 hPa/s.

Préférentiellement, la pression (P) continue de croître jusqu'à ce qu'elle atteigne une valeur de pression maximale (Pₘₐₓ). Préférentiellement, la pression (P) appliquée par l'appareil est égale à la pression maximale (Pₘₐₓ) jusqu'à ce que une période de temps (TimeOut) soit écoulée ou jusqu'à ce que le débit mesuré (D) soit supérieur au débit d'air minimal à atteindre en phase inspiratoire (Dₘᵢₙ(insp)) ou jusqu'à ce que le débit d'air mesuré soit inférieur au débit d'air de base (D_{base}).

Préférentiellement, la pression (P) appliquée par l'appareil décroît dès qu'une période de temps (TimeOut) est écoulée ou dès que le débit mesuré (D) est supérieur au débit d'air minimal à atteindre en phase inspiratoire (Dₘᵢₙ(insp)) ou dès que le débit d'air mesuré est inférieur au débit d'air de base (D_{base}). Préférentiellement, lorsque ladite pression (P) décroît, la pression décroît jusqu'à une valeur de pression de base (P_{base}), le procédé étant ensuite initialisé et appliqué à nouveau, ceci permettant une nouvelle croissance de ladite pression (P) lors de chaque phase inspiratoire.

L'invention sera mieux comprise à la lecture de l'exposé détaillé non limitatif qui suit, rédigé au regard des dessins annexés, dans lesquels :
- la figure 1 montre, en perspective, un appareil d'assistance respiratoire à distribution d'air sous pression positive continue selon l'invention ;
- la figure 2 présente des courbes montrant les variations du débit d'air et de la pression en fonction du temps, selon l'invention ;
- les figures 3A et 3B illustrent le décalage de variation du débit mesuré dû aux fuites d'air ; et
- les figures 4A et 4B illustrent les différentes étapes du procédé mis en oeuvre par l'appareil selon l'invention.

Les appareils CPAP 1 selon l'invention se présentent sous la forme d'un boîtier 2 relié, par un tube 3, à un masque 4 destiné à recouvrir le nez et la bouche d'un patient 5.

Le boîtier 2 est un boîtier portable muni d'un écran 6 et de boutons poussoirs 7. Il comporte une turbine non représentée sur les figures. Cette turbine est actionnée en rotation par un moteur, pour envoyer de l'air sous pression, par le tube 3, jusqu'au masque 4. La rotation de la turbine est commandée par une électronique incorporée au boîtier 2. Cette électronique comprend en particulier une carte électronique portant des mémoires de type volatile et non volatile (EEPROM ou Flash) et un microcontrôleur.

Le masque 4 est un masque facial comportant notamment un orifice 8. Alternativement, un masque nasal peut également être utilisé. L'air respiré par le patient 5, chargé en dioxyde de carbone, s'échappe essentiellement par cet orifice 8.

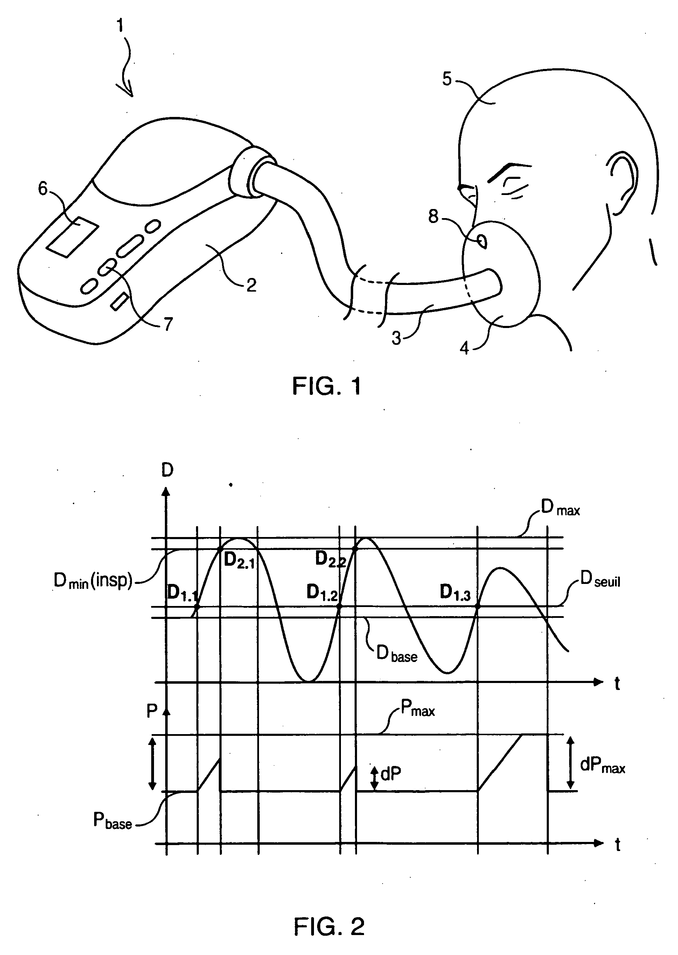

La figure 2 est un graphe qui présente deux courbes superposées. La première courbe montre les variations de débit d'air mesurées D par l'appareil 1 en fonction du temps t. La seconde courbe montre les variations de pression P appliquées par ledit appareil en fonction du temps t.

La première courbe D = f(t) se présente sous une forme sensiblement sinusoïdale, qui traduit le cycle respiratoire du patient. En phase inspiratoire, le débit mesuré augmente tandis qu'en phase expiratoire, il diminue. Différentes valeurs remarquables de débit ont été figurées, sur le graphe, par des lignes horizontales. Il s'agit d'une valeur de débit de base D_{base}, d'une valeur seuil de débit Dₛₑᵤᵢₗ, d'une valeur minimale de débit à atteindre en phase inspiratoire Dₘᵢₙ(insp) et d'une valeur maximale de débit Dₘₐₓ. On a D_{base} < Dₛₑᵤᵢₗ < Dₘᵢₙ(insp) < Dₘₐₓ. Par ailleurs, Dₘᵢₙ(insp) est fixé comme étant un pourcentage de Dₘₐₓ. Avantageusement, Dₘᵢₙ(insp) est compris entre 70 et 90 % de Dₘₐₓ. Par exemple, Dₘᵢₙ(insp) = 80% de Dₘₐₓ. Sur la figure 2, la courbe D est représentée sur trois cycles d'inspiration/expiration.

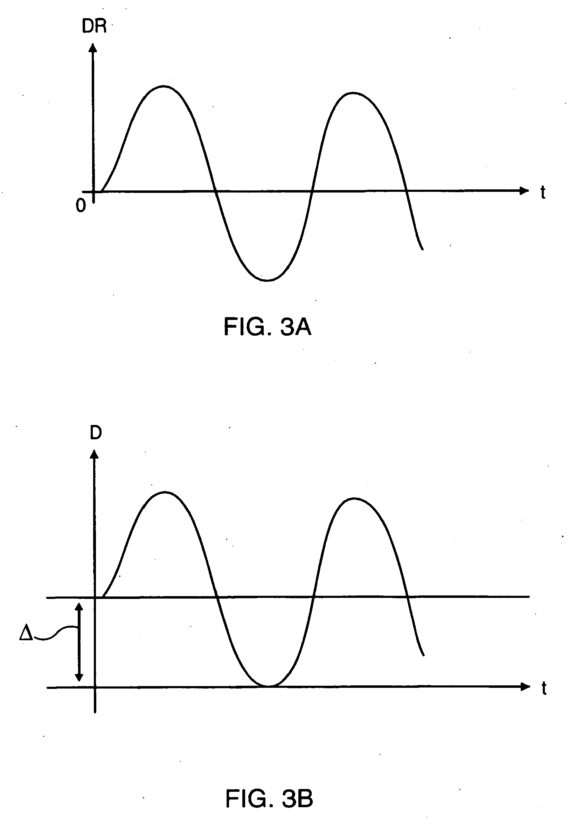

On notera, à l'appui des courbes présentées aux figures 3A et 3B, que le débit d'air effectif appliqué aux voies aériennes supérieures du patient, noté DR à la courbe de la figure 3A est différent du débit d'air mesuré D présenté à la figure 3B. En effet, il y a un décalage de débit dû à l'air s'échappant par l'orifice 8, ce décalage étant référencé Δ à la figure 3B.

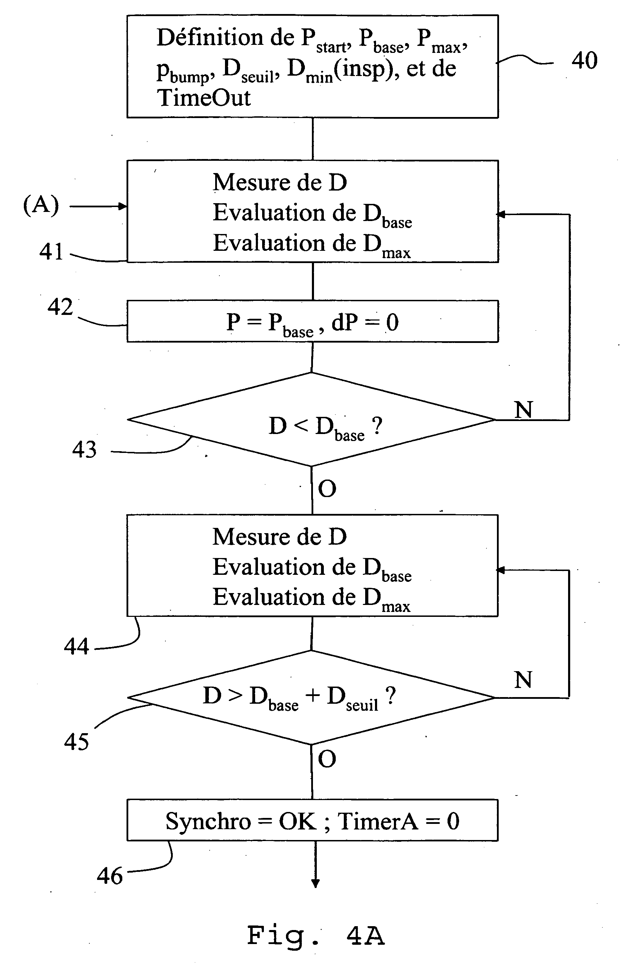

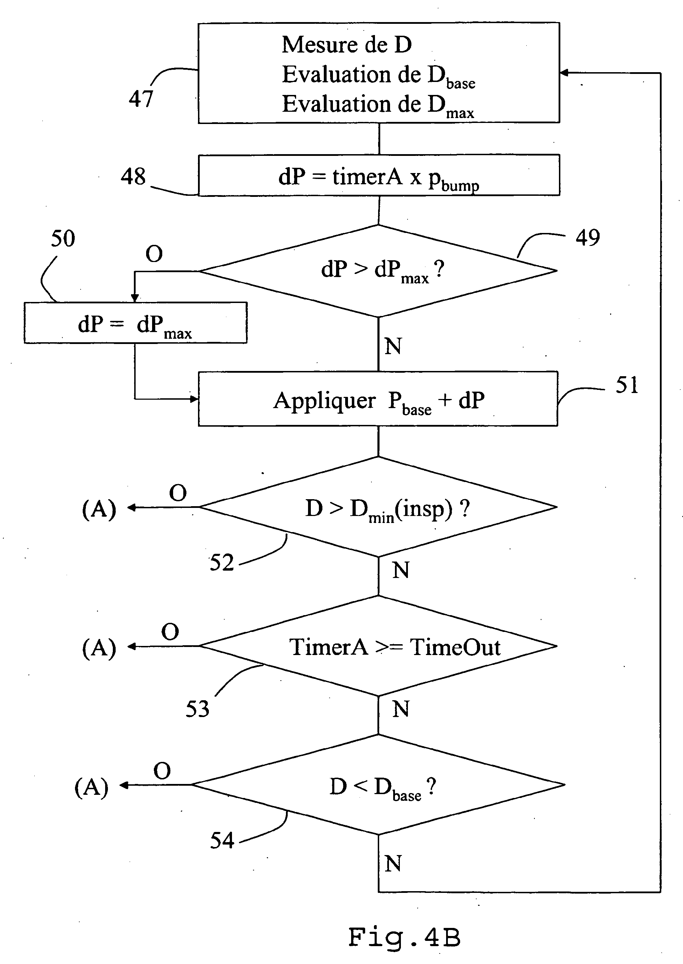

En figure 2, la seconde courbe P = f(t) est une courbe sensiblement constante telle que P = P_{base} où P_{base} est la pression de base délivrée par l'appareil correspondant à la pression minimale à appliquer au patient. Cette courbe présente cependant des protubérances, ces protubérances étant formées par des périodes de croissance de pression sensiblement linéaires suivies de diminutions brutales de pression jusqu'à la pression P_{base}. De même que pour la première courbe, la seconde courbe montre des valeurs remarquables. Il s'agit d'une valeur de la pression précitée P_{base}, et d'une valeur maximale Pₘₐₓ à appliquer audit patient. Il est à noter que les figures 2, 4A et 4B représentent un mode de réalisation préférentiel, où les périodes de croissance de pression sont linéaires ou sensiblement linéaires, c'est-à-dire où P=f(t) est une fonction linéaire. Il est toutefois possible d'envisager que P=f(t) soit une fonction croissante non linéaire.

En pratique, en phase inspiratoire, lorsque D_{base} ≤ D ≤ Dₛₑᵤᵢₗ, P est égal à P_{base}. Toutefois, lorsque, toujours en phase inspiratoire, D atteint Dₛₑᵤᵢₗ (respectivement les points D_{1.1}, D_{1.2} et D_{1.3} pour le premier, deuxième et troisième cycle inspiration/expiration sur la figure 2), alors P croît, préférentiellement de manière linéaire selon une pente p_{bump} comprise entre 0,05 et 20 hPa/s, jusqu'à ce que D atteigne la valeur Dₘᵢₙ(insp) (points D_{2.1} et D_{2.2}). Préférentiellement, p_{bump} est égale à 0.6, 1.2, ou 2.0 hPa/s. Cette pression croissante délivrée par l'appareil facilite la phase inspiratoire chez le patient. Lorsque D = Dₘᵢₙ(insp), la pression P diminue rapidement, en quelques ms, pour atteindre P_{base}. La pression P reste alors égale à P_{base} en phase expiratoire, jusqu'à la prochaine phase inspiratoire, lorsque, de nouveau, D = Dₘᵢₙ(insp). Si toutefois, comme cela est le cas en partie droite de la figure 2, D n'atteint pas, en phase inspiratoire, Dₘᵢₙ(insp), la pression P continue alors de croître selon la pente précitée p_{bump} jusqu'à atteindre Pₘₐₓ. A ce moment, P reste constante et égale à Pₘₐₓ jusqu'à la fin d'un intervalle de temps TimeOut, ou jusqu'à ce que D soit inférieur à D_{base} ou jusqu'à ce que D soit supérieur à Dₘᵢₙ(insp). Dans le cas de la figure 2, P décroit lorsque D est inférieur à D_{base}.

Les figures 4A et 4B présentent les différentes étapes de mise en oeuvre du procédé selon l'invention. Les étapes de la figure 4A sont relatives à une synchronisation de l'appareil et les étapes de la figure 4B sont relatives à la mise en oeuvre des protubérances de pression selon l'invention.

En pratique, dans une première étape 40 le médecin définit, pour un patient donné, les valeurs suivantes : Pₛₜₐᵣₜ, la pression appliquée par l'appareil à son démarrage ; P_{base} ; Pₘₐₓ ; P_{bump} ; Dₛₑᵤᵢₗ ; Dₘᵢₙ(insp) égale à un pourcentage donné de Dₘₐₓ déterminé ultérieurement ; et TimeOut, le temps maximal durant lequel P peut rester égal à Pₘₐₓ. Ces valeurs sont entrées dans la ou les mémoires non volatiles de l'appareil en utilisant les boutons.

Puis, après que l'appareil ait été mis en fonctionnement, et que le masque ait été appliqué au visage du patient, l'appareil mesure le débit D, évalue le débit D_{base} image du débit de fuite d'air par l'orifice 8 ainsi que le débit Dₘₐₓ, en fonction des maxima de débit atteints en phase inspiratoire (étape 41).

Dans une étape ultérieure 42 P, la pression appliquée par l'appareil, est fixée à P_{base}. Aucune augmentation de pression, dP, n'est appliquée par l'appareil.

L'appareil effectue alors le test 43 suivant : est-ce que D, le débit d'air mesuré, est inférieur à D_{base} ? Dans la négative, on retourne à l'étape 41. Dans l'affirmative, une nouvelle étape 44 de mesure de D et d'évaluation de D_{base} et de Dₘₐₓ est effectuée. Puis, le test suivant 45 est effectué : est ce que D > D_{base} + Dₛₑᵤᵢₗ ou, autrement dit, est ce que le point D1 de la première courbe de la figure 2 a été atteint ? Dans la négative, on retourne à l'étape 44. Dans l'affirmative, la synchronisation est terminée et un registre d'horloge TimerA est fixé à zéro pour s'incrémenter par la suite (étape 46).

Dès que le registre d'horloge est fixé à zéro, les étapes suivantes de la figure 4B sont mise en oeuvre.

Dans une première étape 47, l'appareil mesure D, évalue D_{base} et évalue Dₘₐₓ, tout comme dans l'étape 41 précitée. Puis, dans une étape 48, l'appareil détermine l'écart de pression dP entre la pression P à appliquer et la pression de base P_{base}, tel que dP = TimerA x p_{bump}. Un premier test 49 est alors effectué : est ce que l'écart de pression dP > dPₘₐₓ ? (dPₘₐₓ correspondant à l'écart entre Pₘₐₓ et P_{base}) Dans l'affirmative dP est ramené à dPₘₐₓ (étape 50), puis on passe à l'étape 51, dans laquelle l'appareil applique une pression égale à P_{base} augmentée de dP. C'est-à-dire que la pression à appliquer P est limitée à la pression maximale Pₘₐₓ qui doit être appliquée au patient. Si la réponse au test 49 est négative, on passe directement à l'étape 51. Autrement dit, la pression appliquée P est augmentée graduellement et linéairement selon le facteur p_{bump}.

Ensuite, un second test 52 est effectué. Ce second test consiste à déterminer si la valeur minimum de débit à atteindre en phase inspiratoire a été atteinte, c'est-à-dire, est ce que D > Dₘᵢₙ(insp) ? Dans l'affirmative, on retourne à l'étape 41 de la figure 4A et P est ramené à P_{base} (étape 42). Dans la négative, un troisième test 53 est réalisé selon lequel on détermine si TimerA ≥ TimeOut. Dans l'affirmative, on retourne à l'étape 41. Dans la négative, le quatrième et dernier test suivant 54 est réalisé : est ce que D < D_{base}? Dans l'affirmative, on retourne, comme précédemment, à l'étape 41. Dans la négative, on retourne à l'étape 45 de la présente figure 4B.

Ainsi dans l'exemple de cycle respiratoire représenté en figure 2, l'étape 46 correspond aux points D1.1, D1.2 et D1.3. Une fois ces points atteints, les étapes 47, 48, 49, 51, 52, 53 et 54 sont effectuées en boucle, de sorte que l'on a une augmentation linéaire de la pression de traitement. Pour les premier et deuxième cycles d'inspiration/expiration, ceci est effectué jusqu'à ce que la réponse à l'étape 52 soit affirmative, c'est-à-dire jusqu'à ce que les points D2.1 et D2.2 soient atteints. La pression est alors ramenée à la valeur P_{base}. Les étapes de synchronisation (figure 4A) sont effectuées jusqu'au prochain cycle d'inspiration/expiration. En revanche lors du troisième cycle d'inspiration/expiration, le débit n'atteint jamais la valeur Dₘᵢₙ(insp). La pression est donc augmentée jusqu'à ce que la réponse à l'étape 49 soit affirmative ; la pression est alors plafonnée à la valeur Pmax (P_{base}+dP_{Max}) (étape 50). Puis les étapes 51, 52, 53, 54, 47, 48, 49, 50 sont effectuées en boucles, jusqu'à ce que la réponse à l'étape 54 soit affirmative, ce qui correspond au moment où la courbe D=f(t) atteint le point D_{3.3}. La pression est alors ramenée à P_{base}. L'appareil exécute alors les étapes de synchronisation (figure 4A) jusqu'au prochain cycle d'inspiration/ expiration.

En pratique, le registre d'horloge s'incrémente toutes les 32 ms. Ainsi, les cadences de traitement selon les cycles ci-dessus des figures 4A et 4B sont implémentés toutes les 32 ms, ce qui correspond à un échantillonnage suffisant pour l'usage de l'appareil. Par ailleurs, Dₘₐₓ est évaluée avec une relative certitude au bout de 30 cycles respiratoires. Ainsi, ce n'est qu'au bout de ces 30 cycles que la limite Dₘᵢₙ(insp) sera déterminée avec précision.

En définitive, grâce au procédé selon l'invention, on aide le patient dans les phases inspiratoires en lui fournissant une pression d'air augmentée par rapport à la pression de base. Peu avant la survenance des phases expiratoires, en pratique à Dₘᵢₙ(insp), cette pression diminue brutalement de sorte à limiter les effets négatifs d'une pression trop élevée en phase expiratoire. Cependant, si le débit d'air mesuré en phase inspiratoire s'avère insuffisant, ce qui constitue un signe annonciateur d'un dérèglement du cycle respiratoire susceptible de s'accompagner d'une apnée obstructive en phase expiratoire, alors la pression est augmentée de manière à aider le patient à accomplir une inspiration correcte sans que cette augmentation de pression soit nuisible, et ce, grâce notamment à la prise en compte d'un temps maximum durant lequel une pression maximale peut être maintenue.

Bien entendu, l'invention doit se comprendre dans un cadre large incluant tous les modes de mise oeuvres non exposés dans la présente demande, à la portée de l'homme du métier.

## Revendications

1. Appareil (1) d'assistance respiratoire à distribution d'air sous pression positive continue comportant un boîtier (2) muni d'une turbine actionnée en rotation par un moteur pour envoyer de l'air sous une pression (P) jusqu'à un masque (4), des moyens pour mesurer un débit d'air (D) ainsi qu'une électronique pour une commande de la rotation de ladite turbine, cette électronique comportant une mémoire, ledit appareil (1) étant **caractérisé en ce qu'**il comporte des moyens pour évaluer des valeurs de débit d'air de base (D_{base}) et de débit d'air maximal (Dₘₐₓ) pour un patient, et **en ce qu'**il est apte à délivrer une pression d'air (P) croissante lorsque le débit d'air mesuré (D) n'atteint pas un débit d'air minimal à atteindre dans une phase inspiratoire (Dₘᵢₙ(insp)) défini dans la mémoire de l'électronique de l'appareil (1), puis à délivrer une pression d'air décroissante lorsque ledit débit d'air (D) atteint ledit débit, d'air minimal à atteindre dans ladite phase inspiratoire (Dₘᵢₙ(insp)), la valeur de ce débit d'air minimal à atteindre étant supérieure au débit de base (D_{base}) et inférieure au débit maximal (Dₘₐₓ).

2. Appareil (1) selon la revendication 1, **caractérisé en ce que** la valeur du débit d'air minimal à atteindre (Dₘᵢₙ(insp)) est comprise entre 70 et 90 % de la valeur du débit d'air maximal (Dₘₐₓ).

3. Appareil (1) selon l'une des revendications 1 ou 2, **caractérisé en ce qu'**une une valeur seuil de débit d'air (Dₛₑᵤᵢₗ) est définie dans la mémoire de l'électronique de l'appareil (1), cette valeur seuil étant supérieure à la valeur de base du débit d'air (D_{base}) et inférieure à la valeur du débit minimal à atteindre en phase inspiratoire, et **en ce que** l'appareil (1) comprend des moyens aptes à débuter la délivrance d'une pression d'air (P) croissante, lorsque le débit d'air atteint cette valeur seuil en phase inspiratoire.

4. Appareil (1) selon l'une des revendications précédentes, **caractérisé en ce qu'**il comprend des moyens aptes à délivrer une pression d'air (P) croissante selon une augmentation de pression (P_{bump}) comprise entre 0,05 et 20 hPa/s.

5. Appareil (1) selon l'une des revendications précédentes, **caractérisé en ce qu'**il comprend des moyens aptes à délivrer une pression d'air (P) croissante jusqu'à, ce que ladite pression (P) atteigne une valeur de pression maximale (Pₘₐₓ).

6. Appareil (1) selon la revendication 5, **caractérisé en ce qu'**il comprend des moyens aptes à appliquer une pression (P) égale à la pression maximale (Pmax) jusqu'à ce que une période de temps (TimeOut) soit écoulée ou jusqu'à ce que le débit mesuré (D) soit supérieur au débit d'air minimal à atteindre en phase inspiratoire (Dₘᵢₙ(insp)) ou jusqu'à ce que le débit d'air mesuré soit inférieur au débit d'air de base (D_{base}).

7. Appareil (1) selon l'une des revendications précédentes, **caractérisé en ce qu'**il comprend des moyens aptes à appliquer une pression (P) décroissante dès qu'une période de temps (TimeOut) est écoulée ou dès que le débit mesuré (D) est supérieur au débit d'air minimal à atteindre en phase inspiratoire (Dₘᵢₙ(insp)) ou dès que le débit d'air mesuré est inférieur au débit d'air de base (D_{base}).

8. Appareil (1) selon la revendication 7, **caractérisé en ce que** lorsque ladite pression (P) est décroissante, elle décroît jusqu'à une valeur de pression de base (P_{base}), et **en ce que**, à une telle pression de base, l'appareil comprend des moyens d'initialisation, ceci permettant une nouvelle croissance de ladite pression (P) lors de chaque phase inspiratoire.

## Claims

1. A breathing aid air distribution device (1) for distributing air under continuous positive pressure, comprising a box (2) provided with a rotation-activated turbine via a motor for sending the air under a pressure (P) to a mask (4), means for measuring an air rate (D) as well as an electronic device for controlling the rotation of said turbine, this electronic device comprising a memory, said air distribution apparatus (1) being **characterised in that** it comprises evaluation means for evaluating a basic air rate value (D_{base}) and a maximum air rate value (Dₘₐₓ) for a patient, and **in that** it is able to deliver a rising air pressure (P) when the measured air rate (D) does not reach a minimum air rate to be reached in an inhaling phase (Dₘᵢₙ(insp)) defined in the memory of the electronic device in the air distribution device (1), thereafter to deliver a falling air pressure when said air rate (D) reaches said minimum air rate to be reached in said inhaling phase (Dₘᵢₙ(insp)), the value of this minimum air rate to be reached being higher than the basic rate (D_{base}) and lower than the maximum rate (Dₘₐₓ)

2. An air distribution device as claimed in claim 1, **characterised in that** the value of the minimum air rate to be reached (Dₘᵢₙ(insp)) is comprised between 70 and 90% of the value of the maximum air rate (Dₘₐₓ)

3. An air distribution device as claimed in claim 1 or 2, **characterised in that** an air rate threshold value (Dₛₑᵤᵢₗ) is defined in the memory of the electronic device of the air distribution device (1), this threshold value being higher than the basic value of the air rate (D_{base}) and lower than the value of the minimum rate to be reached in the inhaling phase, and **in that** the air distribution device (1) comprises means able to start the delivery of a rising air pressure (P) when the air rate reaches said threshold value in the inhaling phase.

4. An air distribution device as claimed in any one of the preceding claims, **characterised in that** it comprises means able to deliver a rising air pressure (P) in accordance with a rise in pressure (P_{bump}) comprised between 0.05 and 20 hPa/s.

5. An air distribution device as claimed in any one of the preceding claims, **characterised in that** it comprises means able to deliver a rising air pressure (P) until said pressure (P) reaches a maximum pressure value (Pₘₐₓ).

6. An air distribution device as claimed in claim 5, **characterised in that** it comprises means able to apply a pressure (P) that is equal to the maximum pressure (Pₘₐₓ) until a period of time (TimeOut) has elapsed or until the measured rate (D) is higher than said minimum air rate to be reached in the inhaling phase (Dₘᵢₙ(insp)) or until the measured air rate is lower than the basic air rate (D_{base}).

7. An air distribution device as claimed in any one of the preceding claims, **characterised in that** it comprises means able to apply a falling pressure (P) the moment a period of time (TimeOut) has elapsed or the moment the measured rate (D) is higher than the minimum air rate to be reached in the inhaling phase (Dₘᵢₙ(insp)) or the moment the measured air rate is lower than the basic air rate (D_{base}).

8. An air distribution device (1) as claimed in claim 7, **characterised in that** when said pressure (P) is falling, it falls to a basic pressure value (P_{base}), and **in that** at such basic pressure the air distribution device comprises initialization means which allows of a new rise of said pressure (P) during each inhaling phase.

## Patentansprüche

1. Vorrichtung (1) zur Unterstützung der Atmung mit einer Luftverteilung mit kontinuierlich positivem Atemwegsdruck (CPAP) mit einem Gehäuse (2), das mit Folgendem ausgestattet ist: einer von einem Motor in einer Drehbewegung angetriebenen Turbine, um Luft unter einem Druck (P) einer Maske (4) zuzuführen, Mitteln zum Messen der Luftmenge (D) sowie einer Elektronik für die Steuerung der Drehbewegung der genannten Turbine, wobei diese Elektronik einen Speicher umfasst, wobei die genannte Vorrichtung (1) **dadurch gekennzeichnet ist, dass** sie Mittel zum Auswerten der Werte der Basisluftmenge (D_{base}) und der Maximalluftmenge (Dₘₐₓ) für einen Patienten umfasst und dass sie geeignet ist, einen zunehmenden Luftdruck (P) zu liefern, wenn die gemessene Luftmenge (D) die in einer Inspirationsphase zu erreichende Mindestluftmenge (Dₘᵢₙ(insp)), die in dem Speicher der Elektronik der Vorrichtung (1) festgelegt ist, nicht erreicht, danach einen abnehmenden Luftdruck zu liefern, wenn die genannte Luftmenge (D) die in der genannten Inspirationsphase zu erreichende genannte Mindestluftmenge (Dₘᵢₙ(insp)) erreicht, wobei der Wert dieser zu erreichenden Mindestluftmenge höher als die Basisluftmenge (D_{base}) und niedriger als die Maximalluftmenge (Dₘₐₓ) ist.

2. Vorrichtung (1) nach Anspruch 1, **dadurch gekennzeichnet, dass** der Wert der zu erreichenden Mindestluftmenge (Dₘᵢₙ(insp)) zwischen 70 und 90% des Wertes der Maximalluftmenge (Dₘₐₓ) liegt.

3. Vorrichtung (1) nach einem der Ansprüche 1 oder 2, **dadurch gekennzeichnet, dass** in dem Speicher der Elektronik der Vorrichtung (1) ein Schwellenwert der Luftmenge (Dₛₑᵤᵢₗ) festgelegt wird, wobei dieser Schwellenwert höher als der Basiswert der Luftmenge (D_{base}) und niedriger als der Wert der in der Inspirationsphase zu erreichenden Mindestluftmenge ist, und dass die Vorrichtung (1) Mittel umfasst, die geeignet sind, die Lieferung eines zunehmenden Luftdrucks (P) zu starten, wenn die Luftmenge in der Inspirationsphase diesen Schwellenwert erreicht.

4. Vorrichtung (1) nach einem der vorhergehenden Ansprüche, **dadurch gekennzeichnet, dass** sie Mittel umfasst, die geeignet sind, einen zunehmenden Luftdruck (P) gemäß einer Druckzunahme (p_{bump}) zwischen 0,05 und 20hPA/s zu liefern.

5. Vorrichtung (1) nach einem der vorhergehenden Ansprüche, **dadurch gekennzeichnet, dass** sie Mittel umfasst, die geeignet sind, einen zunehmenden Luftdruck (P) zu liefern, bis der genannte Druck (P) einen Maximalwert des Druckes (Pₘₐₓ) erreicht.

6. Vorrichtung (1) nach Anspruch 5, **dadurch gekennzeichnet, dass** sie Mittel umfasst, die geeignet sind, einen dem Maximaldruck (Pₘₐₓ) entsprechenden Druck (P) anzuwenden, bis eine Zeitspanne (TimeOut) verstrichen ist oder die gemessene Menge (D) über der in der Inspirationsphase zu erreichenden Mindestluftmenge (Dₘᵢₙ(insp)) liegt oder die gemessene Luftmenge unter der Basisluftmenge (D_{base}) liegt.

7. Vorrichtung (1) nach einem der vorhergehenden Ansprüche, **dadurch gekennzeichnet, dass** sie Mittel umfasst, die geeignet sind, einen abnehmenden Druck (P) anzuwenden, sobald eine Zeitspanne (TimeOut) verstrichen ist oder sobald die gemessene Menge (D) über der in der Inspirationsphase zu erreichenden Mindestluftmenge (Dₘᵢₙ(insp)) liegt oder sobald die gemessene Luftmenge unter der Basisluftmenge (D_{base}) liegt.

8. Vorrichtung (1) nach Anspruch 7, **dadurch gekennzeichnet, dass** wenn der genannte Druck (P) abnimmt, er bis zu einem Basiswert des Druckes (P_{base}) abnimmt, und dass bei einem derartigen Basisdruck die Vorrichtung Mittel zur Initialisierung umfasst, die eine erneute Zunahme des genannten Drucks (P) bei jeder Inspirationsphase ermöglicht.
